# EUROPÄISCHE PATENTSCHRIFT

(11) **EP 0 337 053 B1**
(45) Veröffentlichungstag und Bekanntmachung des Hinweises auf die Patenterteilung: **26.04.1995**
(21) Anmeldenummer: 89100380.8
(22) Anmeldetag: 11.01.1989
(51) Int. Cl.: G01N 33/52

(54) **Teststreifen zur Bestimmung der Sauerstoffkonzentration in einer Flüssigkeit sowie Verfahren zu seiner Herstellung.**
Teststrip to determine the oxygen concentration in a fluid and method to prepare it
Bande d'analyse pour déterminer la concentration d'oxygène dans un liquide et méthode de préparation

(30) Priorität: 13.01.1988 DE 3800661
(43) Veröffentlichungstag der Anmeldung: 18.10.1989
(73) Patentinhaber: Verein der Zuckerindustrie, D-53113 Bonn (DE)
(72) Erfinder: Buchholz, Klaus, Dr., D- 3300 Braunschweig (DE); Mikhael, Issam, Dipl.Chem., Langer Kamp 5, D- 3300 Braunschweig (DE)
(74) Vertreter: Einsel, Martin, Dipl.-Phys.

(56) Entgegenhaltungen:
- DE-A- 1 673 307
- GB-A- 1 083 683
- CHEMICAL ABSTRACTS, Band 109, 1988, Columbus, OH (US); K. BUCHHOLZ et al., Nr. 225797j/
- ANALYTICAL CHEMISTRY, Band 46, Nr. 5, April 1974, US; M.M. FISHMAN et al., Seiten 367R-393R/
- CHEMICAL ABSTRACTS, Band 101, 1984, Columbus, OH (US); V. PACAKOVA et al., Seite 302, Nr. 68584c/
- BIOLOGICAL ABSTRACTS, Band 73, 1982, Biological Abstracts Inc., Philadelphia, PA (US); V.L. BAZELYAN et al., Nr. 30873/
- CHEMICAL ABSTRACTS, Band 102, 1985, Columbus, OH (US); E. VILANOVA et al., Seite 272, Nr. 58393w/
- BIOLOGICAL ABSTRACTS, Band 80, 1985, Biological Abstracts Inc., Philadelphia, PA (US); MADHL et al., Nr. 48856/

## Beschreibung

Die Erfindung betrifft einen Teststreifen aus einem saugfähigen Werkstoff oder mit einer saugfähigen Beschichtung aus diesem Werkstoff, insbesondere aus Zellulose oder Kunststoff sowie ein Verfahren zur Herstellung derartiger Teststreifen.

Es sind Teststreifen aus einem saugfähigen Werkstoff oder mit einer saugfähigen Beschichtung aus Zellulose- oder Kunststoffasern bekannt, die als Indikatoren für bestimmte Inhaltstoffe von Flüssigkeiten verwendet werden. Insbesondere für die pH-Wertmessung werden derartige Teststreifen benutzt, die sich je nach dem pH-Wert der Flüssigkeit unterschiedlich bzw. unterschiedlich stark verfärben.

Der Erfindung liegt die Aufgabe zugrunde, einen Teststreifen zu schaffen, mit dem es möglich ist, die Sauerstoffkonzentration in Flüssigkeiten zu bestimmen. Zur bisherigen Ermittlung der Sauerstoffkonzentrationen in wässrigen Systemen sind relativ aufwendige Verfahren erforderlich, für die eine Probeentnahme notwendig ist, welche labormäßig untersucht werden muß oder für die aufwendige Meßgeräte eingesetzt werden müssen. Zur schnellen Bestimmung der Sauerstoffkonzentration in Flüssigkeiten, insbesondere in der Wasser- und Abwassertechnik sind Messungen vor Ort auch durch Fachunkundige sehr erwünscht und zur Registrierung sich verändernder Sauerstoffkonzentrationen, beispielsweise in Anlagen für die Behandlung von Abwassern erforderlich.

Diese Aufgabe wird gelöst durch einen Teststreifen aus einem saugfähigen Werkstoff oder mit einer saugfähigen Beschichtung aus diesem Werkstoff, insbesondere aus Zellulose oder Kunststoff, zum Eintauchen in eine Flüssigkeit, der zur Bestimmung der Sauerstoffkonzentration in der Flüssigkeit mit zum einen Tyrosin oder Dopa und zum anderen immobilisierter Tyrosinase versehen ist, wobei die Tyrosinase pflanzlichen Ursprungs ist und die Immobilisierung durch eine feindisperse Beschichtung oder durch Imprägnierung mit an Bentonit absorbierter Tyrosinase gewährleistet ist.

Dieser Teststreifen ist relativ einfach herstellbar und kann als sehr guter Indikator verwendet werden, da er sich in Abhängigkeit von der Sauerstoffkonzentration der zu messenden Flüssigkeit verfärbt und in Abhängigkeit von dieser Konzentration eine rosa bis braune Farbe annimmt. Anhand einer durch Eichmessungen hergestellten Farbskala kann der Sauerstoffgehalt in wässrigen Systemen auch von Fachunkundigen geprüft und registriert werden.

Die Erfindung beruht auf der Erkenntnis, daß sich Tyrosinase aus pflanzlichen Extrakten oder zerkleinerten Pflanzen durch Schichtmineralien, wie Bentonit, mit hoher Effizienz gewinnen läßt, wobei überraschenderweise die isolierten Enzyme an das Adsorbens in aktiver Form gebunden bleiben.

Bei einem mit Tyrosin bzw. Dopa und Tyrosinase ausgerüsteten Teststreifen wirkt die Tyrosinase in Anwesenheit von Sauerstoff als Katalysator und führt dazu, daß Tyrosin bzw. Dopa zu Farbstoffen umgesetzt werden.

Der Teststreifen muß dabei einen saugfähigen Werkstoff aufweisen, auf welchen die im Anspruch 1 genannten Substanzen haften bleiben und der beim Eintauchen in Flüssigkeit das Eindringen der Flüssigkeit gestattet, damit die Reaktion mit dem in der Flüssigkeit vorhandenen Sauerstoff erfolgen kann. Der Teststreifen kann dabei eine Beschichtung aus den genannten Substanzen bzw. eine Mischung dieser Substanzen aufweisen oder aber mit den genannten Substanzen imprägniert sein.

Als besonders zweckmäßig hat es sich erwiesen, wenn die an Bentonit gebundene Tyrosinase mit einer Puffersubstanz, insbesondere einem Natrium-Phosphatpuffer gemäß Anspruch 2 versetzt wird. Statt Natrium kann auch Kaliumsulfat in der Puffersubstanz Verwendung finden.

Da der Teststreifen auch mit dem Sauerstoff der Atmosphäre reagiert, ist es zweckmäßig, wenn er mit einer den Sauerstoffzutritt verhindernden Umhüllung bzw. Abdeckung versehen ist. Die Umhüllung bzw. der von der Abdeckung umschlossene Raum ist dabei zweckmäßig mit Stickstoff gefüllt.

Ein besonders geeignetes Verfahren zur Herstellung des erfindungsgemäßen beschriebenen Teststreifen besteht darin, daß eine Suspension aus Bentonit und Wasser oder aus Bentonit und einem Natrium-Phosphatpuffer (pH5 mit 0,01 mol/l NaCl) im Verhältnis 1:100 aufgeschlämmt wird, daß Natrium-Phosphatpuffer im Verhältnis von 1:1 mit einem tyrosinasehaltigen Pflanzenextrakt oder zerkleinerten tyrosinasehaltigen Pflanzen unter Stickstoff bei Kühlung auf etwa 4°C vermischt und filtriert wird, daß das Filtrat einem Teil der vorgenannten Suspension im Verhältnis von etwa 2,5:1 bei ca. 4°C verrührt und nach etwa 10 Minuten zentrifugiert und der dabei gewonnenen Feststoff mit wenigstens der 10fachen Menge (bezogen auf das Trockengewicht) Wasser oder dem Natrium- Phosphatpuffer suspendiert und auf einen Streifen aus Zellulose oder Kunststoff bzw. einem Träger mit einer saugfähigen Beschichtung aus Zellulose oder Kunststoff aufgetragen wird, und daß vor oder nach dem Auftrag der suspendierten Feststoffe auf den Streifen Dopa oder Tyrosin in feinverteilter Form aufgebracht wird.

Besonders zweckmäßige Verfahren zur Herstellung der beschriebenen Teststreifen sind in den Ansprüchen 5 bis 8 wiedergegeben.

Durch die bevorzugt verwendete Puffersubstanz wird sichergestellt, daß im Reaktionsbereich des Teststreifens der pH-Wert in der Nähe des Optimums für das Enzym, d.h. um pH 7 liegt.

Nachstehend werden zwei Beispiele für die Gewinnung der Tyrosinase und die Herstellung der Teststreifen beschrieben.

### Beispiel 1:

1g Bentonit (Präparat der Südchemie, BW 100, oder von Sigma B-3378) wird in 100 ml Natrium-Phosphatpuffer (pH 5, mit 0,01 mol/l NaCl) aufgeschlämmt und die Suspension etwa 12 Stunden gerührt.

100 g frisches, in Würfel geschnittenes, zuvor auf ca. 4°C gekühltes Zuckerrübenmaterial wird mit 100 ml gekühltem Phosphatpuffer (pH 5, 0,05 mol/l) in einen Mixer gegeben, in den Stickstoff eingeleitet wird, und dann 2 Minuten im Kühlraum bei ca. 4°C zerkleinert. Der Brei wird anschließend auf einer Nutsche bei Wasserstrahlvakuum filtriert, wobei sich etwa 140 ml Filtrat mit gelöster Tyrosinase ergeben.

250 ml des vorstehend gewonnenen Filtrates werden zu 100 ml der eingangs genannten gekühlten Suspension gegeben und 10 bis 20 Minuten im Kühlraum gerührt, wobei die Adsorption des Enzyms an das Bentonit erfolgt. Anschließend wird etwa 15 Minuten in einer Kühlzentrifuge bei etwa 13.000 g zentrifugiert. Der abgesetzte Feststoff wird dann in 20 ml Phosphatpuffer (pH 5, 0,05 mol/l) suspendiert. Die Suspension kann tiefgefroren aufbewahrt werden.

0,2 ml der Suspension werden auf einem Filterpapierstreifen (1x5 cm²) auf einer Fläche von 1x1 cm² verteilt aufgebracht. Die Ränder dieser Fläche werden mit wenig gesättigter Maltodextrinlösung beschichtet und ein zweiter dünner Papiervliesstreifen auf den ersten gelegt, wobei die Maltodextrinlösung als Kleber dient.

Auf diese Weise hergestellte Teststreifen werden gefriergetrocknet und sind dann mit dem eingeschlossenen Enzym-Bentonit-Komplex lagerfähig.

Zum Testen werden die Streifen zunächst in ein mit Stickstoff begastes Gefäß gegeben (15 Minuten), dann schnell in Lösungen von Dopa mit unterschiedlichen Sauerstoffkonzentrationen getaucht.

In sauerstofffreien Lösungen wird keine Veränderung beobachtet. In Dopalösungen, die mit Luft-Sauerstoff gesättigt sind, entwickelt sich innerhalb einer halben Minute eine schwache, nach 2 Minuten eine deutliche Rosafärbung, die nach etwa 5 Minuten konstant bleibt.

Auf diese Weise läßt sich einwandfrei das Vorhandensein von niedrigen, mittleren oder hohen (auf Sättigung mit Luft bezogenen) Sauerstoffkonzentrationen in wässerigen Phasen ermitteln.

Das im vorgenannten Beispiel gewonnene Filtrat mit gelöster Tyrosinase zeigte gemäß dem am Schluß genannten Test 1 mit Tyrosin als Substrat 26 mU je ml, und gemäß dem ebenfalls am Schluß genannten Test 2 mit Dopa 0,22 U je ml.

Die zum Auftrag auf den Filterpapierstreifen hergestellte Suspension zeigte folgende Aktivitäten:
0,2 ml der Suspension mit 20 ml Tyrosin-Lösung gemäß Test 1 125 mU je ml, mit 20 ml Dopa-Lösung (gemäß Test 2) 1,35 U je ml.

### Beispiel 2

Aus frischem Zuckerrübenmaterial wurde, wie in Beispiel 1 beschrieben, ein Filtrat mit gelöster Tyrosinase hergestellt.

Im Test 1 ergaben sich 144 mU je ml, gemäß Test 2 ergab sich 0,64 U je ml.

Das gewonnene Enzym wurde wie im Beispiel 1 an Bentonit adsorbiert.

Es ergaben sich Aktivitäten der gewonnenen Suspension mit Tyrosin-Lösung gemäß Test 1 von 123 mU je ml, gemäß Test 2 mit Dopa-Lösung 2,5 U je ml Suspension.

Die gewonnene Suspension mit an Bentonit gebundener Tyrosinase wurde in kleinen Portionen von 0,5 bis 5 ml gefriergetrocknet. 50 mg des Präparates ergaben im Test, und zwar gemäß Test 1, mit 20 ml Tyrosin-Lösung eine Aktivität von 1,03 U je g, gemäß Test 2 ergab sich für 10 mg des Präparates mit 20 ml Dopa-Lösung 13,8 U je g. In dieser Form ist das Präparat im Kühlschrank über mehrere Wochen lagerfähig.

Zur Herstellung der Teststreifen wurden 5, 10, 20 und 50 mg des Tyrosinase-Bentonit-Komplexes auf eine Fläche von etwa 1x1 cm² eines Papierstreifens feinverteilt. Dazu wurden 5, 10, 15 und 20 mg Dopa in fester Form feinverteilt zugegeben. Wie im Beispiel 1 wurde ein dünner Vliesstreifen als Deckschicht aufgeklebt.

Vor dem Test wurden die Streifen in einem mit Stickstoff begasten Gefäß mindestens 15 Minuten aufbewahrt. Dann erfolgte ein schnelles Eintauchen in verschiedene Probegefäße, die entweder sauerstofffreies, halb-oder vollständig sauerstoffgesättigtes Wasser bezogen auf Sättigung der Luft enthielten. Im sauerstofffreien Wasser war keine merkliche Veränderung zu beonbachten. Im sauerstoffgesättigten Wasser entwickelte sich eine schwache rosa Färbung, die nach 5 bis 10 Minuten zu einer deutlichen rosa-braunen Färbung des Teststreifens führte. Im halb-sauerstoffgesättigten Wasser war die Farbtönung schwächer.

Versuche haben gezeigt, daß Teststreifen mit je 10 mg Enzym-Bentonit-Komplex und Dopa beschichtet zu der beschriebenen Färbung führten. Demgemäß sind höhere Mengen des genannten Komplexes für die Bestimmung der Sauerstoffkonzentration in wässerigen Lösungen nicht erforderlich.

Erläuterungen der obengenannten Tests zur Messung der Enzymaktivität.

### Test 1:

0,5 ml tyrosinasehaltiges Filtrat und 1,5 ml Tyrosin-Lösung werden bei Zimmertemperatur gemischt und in Küvetten von 1 cm Schichtdicke gegeben. Die Zunahme der Extinktion bei 480 nm Wellenlänge wurde als Funktion der Zeit gemessen.

Die Tyrosin-Lösung besteht aus 10 mg Tyrosin, welche in 100 ml Phosphatpuffer (pH 6,8; 0,05 mol/l) gelöst wurde.

### Test 2:

0,5 ml tyrosinasehaltiges Filtrat werden mit 1,5 ml Dopa-Lösung gemischt und die Reaktion wie in Test 1 verfolgt.

Die Dopa-Lösung besteht aus 20 mg Dopa, welche in 100 ml Phosphatpuffer wie in Test 1 gelöst wurde.

In den Tests mit dem an Bentonit gebundenen Enzym wird analog verfahren, das Reaktionsgefäß jedoch zusätzlich gerührt. Die Mesung der Extinktion erfolgte in zentrifugierten Proben.

Die Aktivitätseinheit U ist definiert als diejenige Enzymaktivität, die in einer Minute eine Steigerung der Extinktion um 1 bewirkt.

## Patentansprüche

1. Teststreifen aus einem saugfähigen Werkstoff oder mit einer saugfähigen Beschichtung aus diesem Werkstoff, insbesondere aus Zellulose oder Kunststoff, zum Eintauchen in eine Flüssigkeit, der zur Bestimmung der Sauerstoffkonzentration in der Flüssigkeit mit zum einen Tyrosin oder Dopa und zum anderen immobilisierter Tyrosinase versehen ist, wobei die Tyrosinase pflanzlichen Ursprungs ist und die Immobilisierung durch eine feindisperse Beschichtung oder durch Imprägnierung mit an Bentonit absorbierter Tyrosinase gewährleistet ist.

2. **Teststreifen nach Anspruch 1,**
**dadurch gekennzeichnet,**
daß die an Bentonit gebundene Tyrosinase mit einer Puffersubstanz, insbesondere einem Natrium-Phosphatpuffer, versetzt ist.

3. Teststreifen nach Anspruch 1 oder 2,
**dadurch gekennzeichnet,**
daß er mit einer vor seiner Benutzung wenigstens teilweise entfernbaren, den Sauerstoffzutritt verhindernden Umhüllung oder Abdeckung versehen ist.

4. Verfahren zur Herstellung von Teststreifen nach einem der Ansprüche 1 bis 3,
**dadurch gekennzeichnet,**
daß eine Suspension aus Bentonit und Wasser oder aus Bentonit und einem Natrium-Phosphatpuffer (pH5 mit 0,01 mol/l NaCl) im Verhältnis 1:100 aufgeschlämmt wird, daß Natrium-Phosphatpuffer im Verhältnis von 1:1 mit einem tyrosinasehaltigen Pflanzenextrakt oder zerkleinerten tyrosinasehaltigen Pflanzen unter Stickstoff bei Kühlung auf etwa 4°C vermischt und filtriert wird, daß das Filtrat mit einem Teil der vorgenannten Suspension im Verhältnis von etwa 2,5:1 bei ca. 4°C verrührt und nach etwa 10 Minuten zentrifugiert und der dabei gewonnene Feststoff mit wenigstens der 10fachen Menge (bezogen auf das Trockengewicht) Wasser oder dem Natrium-Phosphatpuffer suspendiert und auf einen Streifen aus Zellulose oder Kunststoff bzw. einem Träger mit einer saugfähigen Beschichtung aus Zellulose oder Kunststoff aufgetragen wird, und
daß vor oder nach dem Auftrag der suspendierten Feststoffe auf den Streifen Dopa oder Tyrosin in feinverteilter Form aufgebracht wird.

5. Verfahren nach Anspruch 4, **dadurch gekennzeichnet**, daß als zerkleinerte Pflanzen zerkleinerte Zuckerrübenstücke oder als Pflanzextrakt Zuckerrübensaft verwendet werden.

6. Verfahren nach Anspruch 4 oder 5, **dadurch gekennzeichnet**, daß auf eine Fläche von 1 cm² des Teststreifens 5 bis 50 mg des suspendierten Feststoffes aufgebracht wird.

7. Verfahren nach einem der Ansprüche 4 bis 6, **dadurch gekennzeichnet**, daß auf eine Fläche von 1 cm² des Teststreifens 5 bis 30 mg Dopa oder Tyrosin aufgebracht werden.

8. Verfahren nach einem der Ansprüche 4 bis 7, **dadurch gekennzeichnet**, daß wenigstens der beschichtete Teil des Abdeckstreifens mit einer den Sauerstoffzutritt verhindernden Umhüllung versehen bzw. in einer solchen Umhüllung untergebracht wird.

## Claims

1. Test strip consisting of an absorbent material or with an absorbent coating consisting of this material, in particular of cellulose or plastic, for immersing in a liquid, which strip is provided firstly with tyrosine or dopa and secondly with immobilized tyrosinase in order to determine the oxygen concentration in the liquid, the tyrosinase being of plant origin and the immobilisation being effected by a finely dispersed coating or by impregnation with tyrosinase, which is absorbed at bentonite.

2. Test strip according to claim 1, characterised in that the tyrosinase which is bonded to bentonite is mixed with a buffer substance, in particular a sodium phosphate buffer.

3. Test strip according to claim 1 or 2, characterised in that it is provided with a wrapping or cover which can be at least partly removed before it is used and prevents oxygen from entering.

4. Method for producing test strips according to any one of claims 1 to 3, characterised in that a suspension of bentonite and water or of bentonite and a sodium phosphate buffer (ph5 with 0.01 mole/litre NaCl) in a ratio of 1 : 100 is reduced to a slurry, that sodium phosphate buffer is mixed in a ratio of 1 : 1 with a plant extract containing tyrosinase or crushed plants containing tyrosinase in nitrogen while being cooled to approximately 4°C and filtered, that the filtrate is stirred with some of the above-mentioned suspension in a ratio of approximately 2.5 : 1 at approximately 4°C and centrifuged after approximately 10 minutes, and the solid thereby obtained is suspended with at least 10 times the quantity (related to the dry weight) of water or the sodium phosphate buffer and applied to a strip of cellulose or plastic or a base with an absorbent coating of cellulose or plastic, and that dopa or tyrosine is applied in a finely dispersed form before or after the suspended solids are applied to the strip.

5. Method according to claim 4, characterised in that crushed sugar-beet pieces are used as the crushed plants or sugar-beet juice is used as the plant extract.

6. Method according to claim 4 or 5, characterised in that 5 to 50 mg of the suspended solid are applied to a 1 cm² area of the test strip.

7. Method according to any one of claims 4 to 6, characterised in that 5 to 30 mg of dopa or tyrosine are applied to a 1 cm² area of the test strip.

8. Method according to any one of claims 4 to 7, characterised in that at least the coated part of the cover strip is provided with or disposed in a wrapping which prevents oxygen from entering.

## Revendications

1. Bande de test composée d'une matière absorbante ou comportant une couche de cette matière absorbante, notamment de la cellulose ou de la matière plastique, destinée à être plongée dans un liquide et munie, d'une part, de tyrosine ou de dopa et, d'autre part, de tyrosinase immobilisée pour déterminer la concentration d'oxygène dans le liquide, la tyrosinase étant d'origine végétale et l'immobilisation étant garantie par un revêtement finement dispersé ou par imprégnation de tyrosinase absorbée dans de la bentonite.

2. Bande de test selon la revendication 1, caractérisée en ce que la tyrosinase liée à de la bentonite est mélangée à un dépresseur, notamment à un dépresseur de phosphate de sodium.

3. Bande de test selon la revendication 1 ou 2, caractérisée en ce qu'elle est munie d'une enveloppe ou d'une protection empêchant la pénétration d'oxygène qui peut être retirée au moins partiellement avant son utilisation.

4. Procédé pour fabriquer des bandes de test selon l'une des revendications 1 à 3, caractérisé en ce qu'une suspension de bentonite et d'eau ou de bentonite et d'un dépresseur au phosphate de sodium (pH 5 avec 0,01 mole de NaCl par litre) est mise en suspension dans un rapport de 1:100, en ce que le dépresseur de phosphate de sodium est mélangé dans un rapport de 1:1 à un extrait végétal contenant de la tyrosinase ou à des plantes contenant de la tyrosinase dans une atmosphère d'azote en étant refroidi à environ 4° C, puis est filtré, en ce que le filtrat est mélangé avec une partie de la suspension citée précédemment dans un rapport d'environ 2,5:1 à 4° C et est centrifugé après 10 minutes environ, en ce que la matière solide ainsi obtenue est mise en suspension avec au moins 10 volumes d'eau (par rapport au poids à sec) ou avec le dépresseur de phosphate de sodium et est appliquée sur une bande en cellulose ou en matière plastique ou sur un support muni d'un revêtement absorbant en cellulose ou en matière plastique, et en ce que avant ou après l'application de la matière solide en suspension, de la dopa ou de la tyrosine est appliquée sur la bande en étant finement répartie.

5. Procédé selon la revendication 4, caractérisé en ce qu'on utilise en tant que plantes broyées des morceaux de betterave sucrière broyés ou en tant qu'extrait végétal, du jus de betterave sucrière.

6. Procédé selon la revendication 4 ou 5, caractérisé en ce qu'on applique 5 à 50 mg de matière solide en suspension sur une surface de 1 cm² de la bande de test.

7. Procédé selon l'une des revendications 4 à 6, caractérisé en ce qu'on applique 5 à 30 mg de dopa ou de tyrosine sur une surface de 1 cm² de la bande de test.

8. Procédé selon l'une des revendications 4 à 7, caractérisé en ce qu'au moins la partie enduite de la bande de test est munie d'une enveloppe empêchant la pénétration d'oxygène ou est logée dans une telle enveloppe.
